# EUROPEAN PATENT APPLICATION

(11) **EP 4 595 883 A1**
(43) Date of publication of application: **06.08.2025**
(21) Application number: 24211610.1
(22) Date of filing: 08.11.2024
(51) Int. Cl.: A61B 5/349, A61B 5/35, A61B 5/363

(54) **ECG ACTIVATION PATTERN CLUSTERING TEMPLATE ANALYSIS**

(30) Priority: 31.01.2024 US 202418428050
(71) Applicant: Biosense Webster (Israel) Ltd., 2066717 Yokneam (IL)
(72) Inventor: SEGEV, Meytal, 2066717 Yokneam (IL); YARNITSKY, Jonathan, 2066717 Yokneam (IL); YANOVICH, Nir, 2066717 Yokneam (IL); REUVENY, Hadar, 2066717 Yokneam (IL); ROM, lyar, 2066717 Yokneam (IL); COHN, Goren, 2066717 Yokneam (IL); JANKELSON, Lior, 2066717 Yokneam (IL)
(74) Representative: Carpmaels & Ransford LLP

(57) **Abstract**

A method includes receiving a set of electrocardiograms (ECG) determined to belong to a given morphologic template indicative of a given type of arrhythmia. Using a location algorithm, a percentage of the ECGs in the set is calculated, that points to a same source location of the given type of arrhythmia. The calculated percentage is compared to a predefined threshold percentage. If the percentage of ECGs is found to exceed the threshold percentage, the source location is reported to a user.

## Description

### FIELD OF THE DISCLOSURE

The present disclosure relates generally to the analysis of electrocardiograms (ECG), and specifically to determining the source location of an arrhythmia by analyzing ECGs.

### BACKGROUND

Some types of ventricle arrhythmias, such as Premature Ventricular Contraction (PVC), can be diagnosed using ECG. A 12-lead ECG, for example, is useful in providing the initial evidence of PVC frequency and is a quality noninvasive tool to determine the PVC source location and preferable access area. When PVC is suspected from either the history or physical examination of the patient, it is useful to continue to perform a lengthy ECG acquisition (e.g., up to a minute) to determine a better sense of PVC frequency and catch the PVC during the recording of all twelve contemporaneous leads to allow for the most accurate morphology assessment.

A method to identify PVC by identifying at least one ECG as belonging to a given morphological template is provided in U.S. Patent 11,730,414 that describes, in one embodiment, a medical system including respective electrodes for application to a body of a subject and a processor. The electrodes are configured to output a set of respective activation signals in response to the electrical activity of the heart of the subject captured over a sequence of heartbeat intervals. The processor is configured to classify a first heartbeat interval of the set of activation signals as a first morphological template, compute a measure of similarity between a second heartbeat interval of the set of activation signals and the first morphological template, group the second heartbeat interval of the set of activation signals in a first morphological group with the first morphological template responsively to the measure exceeding a predefined threshold, and classify the second heartbeat interval of the set of activation signals as a second morphological template responsively to the measure not exceeding the predefined threshold, and repeat the above, mutatis mutandis, for subsequent heartbeat intervals.

Some academic publications provide location algorithms for estimating the source location of an arrhythmia based on non-invasive ECG data, such as 12-lead ECG or Holter. For example, Muzakkir Amir et. al. describe in a paper titled, "Park Algorithm as Predictor of Premature Ventricular Contraction Origin in Three-Dimensional Mapping Electrophysiological Studies," published in the International Journal of General Medicine Vol. 13, pp. 1083-1092 (2020), the validation of the Park algorithm accuracy in the prediction of the location of origin of PVC using a 12-lead ECG both in the case of PVC with and/or without structural heart disease. The researchers found the Park algorithm suitable to be used for determining the location of PVC origin in the right or left heart.

Park et. al. describe their location algorithm in a paper titled, "Using the surface electrocardiogram to localize the origin of idiopathic ventricular tachycardia," which appeared in the journal Pacing Clinical Electrophysiology Vol. 35, Iss. 12, pp. 1516-1527 (2012).

The present disclosure will be more fully understood from the following detailed description of the examples thereof, taken together with the drawings in which:

### BRIEF DESCRIPTION OF THE DRAWINGS

Fig. 1 is a schematic, pictorial illustration of a cardiac system configured for cardiac electrophysiological (EP) sensing, EP signal analysis, and ablation, according to an example of the present disclosure;
Fig. 2 is a schematic figure of a morphological template used for the analysis of 12-lead ECG acquisition indicative of a given type of arrhythmia, according to an example of the present disclosure; and
Fig. 3 is a flow chart that schematically illustrates a method to statistically determine a cardiac location source of an arrhythmia using a morphological template, according to an example of the present disclosure.

### DETAILED DESCRIPTION OF EXAMPLES

### OVERVIEW

Premature ventricular contractions (PVCs) are extra heartbeats that begin in one of the heart's ventricles. These extra beats disrupt the regular heart rhythm, sometimes causing a sensation of a fluttering or a skipped beat in the chest.

PVCs that cause ectopic beats can occur in isolation or in repeated patterns. The occurrence of three or more consecutive PVCs is classified as ventricular tachycardia (VT). One cause of the ectopic beats is re-entrant signaling, where the heartbeat is initiated by the Purkinje fibers rather than the SA node. For example, if one pathway of the Purkinje fibers is blocked, and another path is experiencing slower conduction, this can trigger an ectopic beat on the post-block pathway. In some cases, PVC is therefore treated using ablation of the ventricle tissue location (e.g., related papillary muscle location) causing the ectopic beats.

Correctly estimating the ventricular source location of an arrhythmia using a non-invasive ECG may be used to select the optimal protocol for an invasive treatment, such as catheter ablation. To this end, various location algorithms, such as those described in the background section, were developed to predict the source location of an arrhythmia using non-invasive ECG techniques (e.g., 12-lead, Holter).

However, given the importance of the required decision (e.g., which invasive procedure to perform based on the estimated location), maximizing the level of confidence in the determination of the source location has great merit.

Examples of the present disclosure that are described hereinafter provide a technique to determine the location of the source of an arrhythmia, such as PVC, with a high degree of confidence by analyzing non-invasively acquired ECGs (e.g., 12-lead acquired ECGs, Holter-acquired ECGs). In an example, the disclosed algorithm enables a processor to identify the most repetitive representative 12-lead ECG template of a heartbeat for the PVC. Using statistical analysis on one or more patterns (i.e., morphological templates) enables the processor to verify, to a high level of confidence, the correct tissue source location in the heart.

In one example, a processor receives a set of electrocardiograms (ECGs) determined to belong to a given morphologic template ("a cluster of ECGs)") indicative of a given type of arrhythmia, such as a set of ECGs obtained using the method described in the aforementioned U.S. Patent 11,730,414. The user can determine the threshold (percentage match) for inclusion in such clustering. Using a location algorithm, such as the above-mentioned Perkins algorithm (for PVC), the processor calculates a percentage of ECGs in the given set that points to the same source location of the given type of arrhythmia. Then the processor compares the calculated percentage to a predefined threshold percentage. If the calculated percentage of ECGs is found to exceed the threshold percentage, the processor reports the source to a user.

Since the number of ECGs in the set can amount to more than a hundred, of which a sufficient number of ECGs point to the same source location, a high level of statistical certainty is attainable by this method. For example, if a sufficient percentage of a sufficient number of ECGs, (e.g. several tens of ECGs) point to the same location (source) of PVC, this effectively verifies an accurate PVC source.

### SYSTEM DESCRIPTION

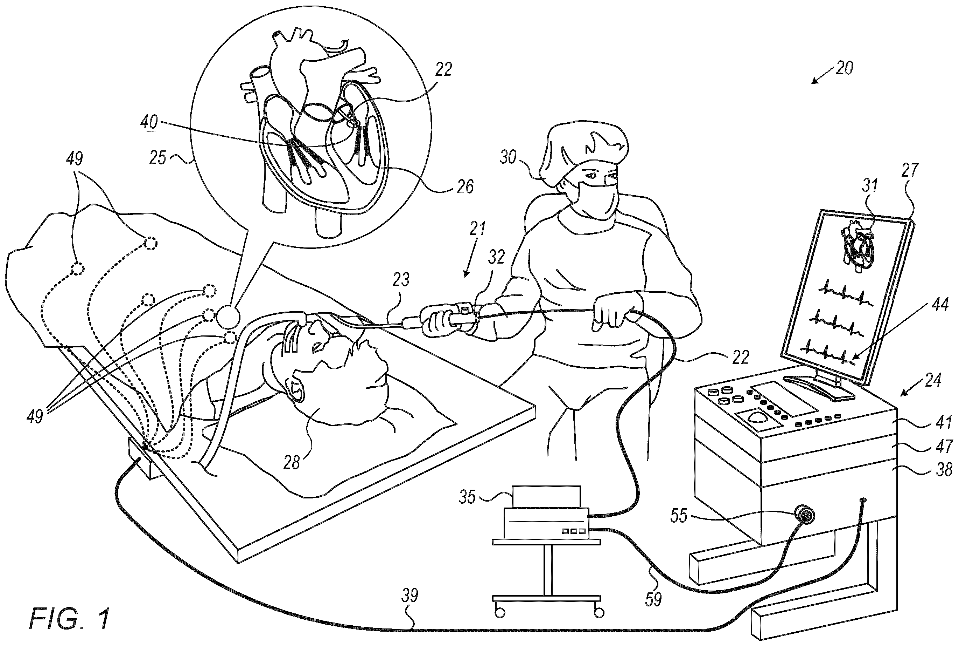

Fig. 1 is a schematic, pictorial illustration of a system 20 configured for cardiac electrophysiological (EP) sensing, EP signal analysis and ablation, according to an example of the present disclosure. System 20 comprises a standalone ECG recorder 35. Recorder 35 is typically used by the physician to view analog ECG signals, such as 12-lead ECG traces (also shown as traces 44 on a display device 27) .

System 20 includes a processing interface unit (PIU) 24, e.g., such as that used by a CARTO^{™} system, produced by Biosense-Webster. ECG leads connected to PIU 24 are sampled for further processing and are also directed to recorder 35 for real-time display of the raw ECG signal. Elements such as electrical power cables, sockets, and inlets are omitted from Fig. 1 for clarity.

As seen, system 20 includes a catheter 21, having a shaft 22 that is navigated by a physician 30 into a heart 26 of a patient 28. In the pictured example, physician 30 inserts shaft 22 through a sheath 23, while manipulating shaft 22 using a manipulator 32 near the proximal end of the catheter.

A distal end 40 of catheter 21 (shown in inset 25) is fitted with electrodes that may be used for pacing, EP mapping, or ablation. The proximal end of catheter 21 is connected to a PIU 24 and to recorder 35, e.g., via PIU 24, e.g., via an output 55 connection of PIU 24.

PIU 24 receives ECG waveforms (e.g., traces) 44 from body surface ECG patches 49. Typically, patches 49 are attached to the skin around the chest and legs of patient 28. PIU 24 is connected to patches 49 by wires running through a cable 39 to receive signals from ECG patches 49. ECG traces 44 are shown on display device 27 (typically at a delay for the same ECG traces shown on recorder 35). Additionally, recorder 35 may receive intra-cardiac signals acquired by the electrodes of catheter 21.

PIU 24 includes processor 41 which may be, for example, a general-purpose computer with a suitable front end and interface circuits 38 for receiving the various signals. In one example of the disclosed technique, processor 41 applies an algorithm to (a) cluster ECG traces 44, and to (b) analyze clustered ECGs acquired over a given number of heartbeats to point at a location (source) of a ventricular arrhythmia, such as PVC.

In another example, processor 41 uses the information contained in intra-cardiac ECG signals obtained using catheter 21 to construct an electrophysiological map 31 and to present it on display device 27.

During an EP mapping procedure, the locations of catheters can be tracked while they are inside heart 26 of the patient. Such tracking may be performed using the Active Current Location (ACL) system, made by Biosense-Webster, which is described in US Patent 8,456,182, whose disclosure is incorporated herein by reference.

Processor 41 may thus associate any given signal received from a catheter, such as intra-cardiac ECGs, with the location at which the signal was acquired. Processor 41 uses information contained in these signals to construct an EP map, such as a local activation time (LAT) map, to present on a display. To perform ablation, electrodes of the catheter may be connected (e.g., switched) to a generator 47.

Processor 41 is typically programmed in software to carry out the functions described herein. The software may be downloaded to the processor in electronic form, over a network, for example, or it may, alternatively or additionally, be provided and/or stored on non-transitory tangible media, such as magnetic, optical, or electronic memory.

### ECG ACTIVATION PATTERN CLUSTERED INTO A MORPHOLOGICAL TEMPLATE

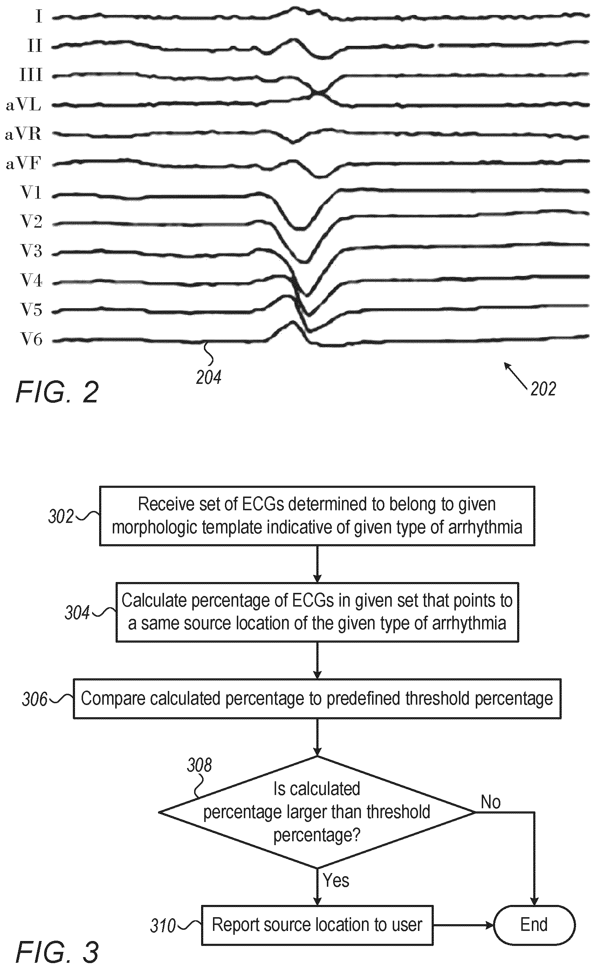

Fig. 2 is a schematic figure of a morphological template 202 used for the analysis of a 12-lead ECG acquisition indicative of a given type of arrhythmia, according to an example of the present disclosure. Fig. 2 shows ECGs 204 acquired using a 12-lead ECG recorder. Morphological template 202 can be used with the method of the above-mentioned U.S. Patent 11,730,414 to acquire a set of ECGs suitable for the statistical analysis method disclosed in this application. For example, assuming a heart rate of 60 BPM, sixty ECG patterns are acquired, of which one portion may represent a normal sinus rhythm and be clustered using a normal sinus rhythm template (not shown), while another portion will fall under the category defined by morphological template 202.

Using the disclosed technique, which, at the first step, applies a location algorithm to the ECGs that fall under morphological template 202, a clinician may statistically assess the confidence level that a given source location is involved in generating abnormal ECGs. Based on the level of confidence, a type of invasive clinical approach may be decided, subject to the clinician's discretion.

### STATISTICALLY DETERMINING A CARDIAC LOCATION SOURCE OF AN ARRHYTHMIA USING A MORPHOLOGICAL TEMPLATE

Fig. 3 is a flow chart that schematically illustrates a method to statistically determine a cardiac location source of an arrhythmia using a morphological template, according to an example of the present disclosure. The process carries out an algorithm that begins with processor 41 receiving a set of ECGs determined to belong to a given morphological template 202 indicative of a given type of arrhythmia, at data receiving step 302.

Next, at a calculation step 304, using a location algorithm, processor 41 calculates a percentage of ECGs in the given set that points to the same source location of the given type of arrhythmia. For example, the processor calculates a fraction of the ECGs that points to a ventricular tissue source location of PVC.

At percentage comparison step 306, the processor compares the calculated percentage to a predefined threshold percentage.

At checking step 308, the processor checks the result of the comparison. If the percentage of ECGs is found to exceed the threshold percentage, the processor reports the source location to a user, at a reporting step 310. The reporting can be a note on display device 27, and/or a highlighted region in a 3D anatomical model shown on the display device.

The flow chart of Fig.3 is brought by way of example and is simplified for clarity of presentation. In another example, the disclosed method is applied, one at a time, to ECG sets collected under a different morphological template.

### EXAMPLES

### Example 1

A method includes receiving a set of electrocardiograms (ECG) determined to belong to a given morphologic template (202) indicative of a given type of arrhythmia. Using a location algorithm, a percentage of the ECGs in the set is calculated, that points to a same source location of the given type of arrhythmia. The calculated percentage is compared to a predefined threshold percentage. If the percentage of ECGs is found to exceed the threshold percentage, the source location is reported to a user (30).

### Example 2

The method according to example 1, wherein the morphologic template (202) is indicative of a Premature Ventricular Contraction (PVC) type of arrhythmia.

### Example 3

The method according to any of examples 1 and 2, wherein reporting the source location comprises reporting an anatomical region within a heart (26).

### Example 4

The method according to any of examples 1 through 3, wherein the set of ECGs was acquired using a 12-lead ECG recorder (35).

### Example 5

A system (20) includes an interface (24) and a processor (41). The interface (24) is configured to receive a set of electrocardiograms (ECG) determined to belong to a given morphologic template (202) indicative of a given type of arrhythmia. The processor (41) is configured to (i) using a location algorithm, calculate a percentage of the ECGs in the set that point to a same source location of the given type of arrhythmia, (ii) compare the calculated percentage to a predefined threshold percentage, and (iii) if the percentage of ECGs is found to exceed the threshold percentage, report the source location to a user (30).

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method, comprising:
receiving a set of electrocardiograms (ECG) determined to belong to a given morphologic template indicative of a given type of arrhythmia;
using a location algorithm, calculating a percentage of the ECGs in the set that point to a same source location of the given type of arrhythmia;
comparing the calculated percentage to a predefined threshold percentage; and
if the percentage of ECGs is found to exceed the threshold percentage, reporting the source location to a user.

2. The method according to claim 1, wherein the morphologic template is indicative of a Premature Ventricular Contraction (PVC) type of arrhythmia.

3. The method according to claim 1, wherein reporting the source location comprises reporting an anatomical region within a heart.

4. The method according to claim 1, wherein the set of ECGs was acquired using a 12-lead ECG recorder.

5. A system, comprising:
an interface configured to receive a set of electrocardiograms (ECG) determined to belong to a given morphologic template indicative of a given type of arrhythmia; and
a processor, which is configured to:
using a location algorithm, calculate a percentage of the ECGs in the set that point to a same source location of the given type of arrhythmia;
compare the calculated percentage to a predefined threshold percentage; and
if the percentage of ECGs is found to exceed the threshold percentage, report the source location to a user.

6. The system according to claim 5, wherein the morphologic template is indicative of a Premature Ventricular Contraction (PVC) type of arrhythmia.

7. The system according to claim 5, wherein the processor is configured to report the source location by reporting an anatomical region within a heart.

8. The system according to claim 5, wherein the set of ECGs was acquired using a 12-lead ECG recorder.
